# EUROPEAN PATENT APPLICATION

(11) **EP 3 245 929 A1**
(43) Date of publication of application: **22.11.2017**
(21) Application number: 15878040.3
(22) Date of filing: 24.12.2015
(51) Int. Cl.: A61B 1/00

(54) **OVER TUBE AND MANIPULATOR SYSTEM**

(30) Priority: 16.01.2015 JP 2015006863
(71) Applicant: Olympus Corporation, Hachioji-shi Tokyo 192-8507 (JP)
(72) Inventor: YANAGIHARA, Masaru, Tokyo 192-8507, (JP); KISHI, Kosuke, Tokyo 192-8507, (JP)
(74) Representative: Schicker, Silvia
(86) International application number: PCT/JP2015/086124
(87) International publication number: WO 2016/114089

(57) **Abstract**

This overtube has a distal-end-side tubular portion (15) that is provided with a first channel (8) through which a manipulator is made to pass and a second channel (9) through which an endoscope is made to pass, and that has flexibility to be driven in accordance with motions of the endoscope; and a proximal-end-side tubular portion (16) that extends the first channel (8) toward a proximal-end side from a proximal end of the distal-end-side tubular portion (15), wherein distal-end openings (20, 21) of the first channel (8) and the second channel (9) are provided at a distal end of the distal-end-side tubular portion (15), a proximal-end opening (22) of the second channel is provided at the proximal end of the distal-end-side tubular portion (15), and a proximal-end opening (23) of the first channel is provided at a proximal end of the proximal-end-side tubular portion (16).

## Description

### {Technical Field}

The present invention relates to an overtube and a manipulator system.

### {Background Art}

In the related art, there is a known overtube having a plurality of channels through which an endoscope and a manipulator are made to pass in an accommodated state (for example, see PTL 1). In this overtube, movable portions that change the shapes of the channels themselves are provided at distal ends of the channels.

### {Citation List}

{Patent Literature}

{PTL 1} Publication of Japanese Patent No. 5052553

### {Summary of Invention}

### {Technical Problem}

When an overtube in which an endoscope channel and a treatment tool channel are provided needs to be inserted into a body by manipulating the overtube or when the position at which treatment is to be performed needs to be changed in the body, providing movable portions in the channels of the overtube, as in the case of PTL 1, makes it necessary to attach/detach driving-force transmitting members, such as wires or the like, which connect the movable portions and driving devices, and thus, there is a problem in which time and work are required to attach/detach the driving-force transmitting members.

The present invention has been made in light of the above-described circumstances, and an object thereof is to provide an overtube and a manipulator system with which it is possible to realize good maneuverability by allowing the overtube to be separated from a driving device without requiring excessive time and effort.

### {Solution to Problem}

In order to achieve the above-described object, the present invention provides the following solutions.

An overtube according to an aspect of the present invention includes: a distal-end-side tubular portion that is provided with a first channel through which a manipulator is made to pass and a second channel through which an endoscope is made to pass, and that has flexibility to be driven in accordance with motions of the endoscope; and a proximal-end-side tubular portion that extends the first channel toward a proximal-end side from a proximal end of the distal-end-side tubular portion, wherein distal-end openings of the first channel and the second channel are provided at a distal end of the distal-end-side tubular portion, a proximal-end opening of the second channel is provided at the proximal end of the distal-end-side tubular portion, and a proximal-end opening of the first channel is provided at a proximal end of the proximal-end-side tubular portion.

With this aspect, the distal end of the manipulator is made to protrude from the distal-end opening of the distal-end-side tubular portion by making the manipulator pass through the first channel of the overtube, and the distal end of the endoscope is made to protrude from the distal-end opening of the distal-end tubular portion by making the endoscope pass through the second channel of the overtube. By doing so, it is possible to perform treatment by using the manipulator while observing the manipulator protruding from the distal-end opening of the overtube by using the endoscope.

In this situation, when the distal-end-side tubular portion is driven in accordance with the motion of the endoscope, the overtube is driven by utilizing the motion of the endoscope even if its own movable portion is not provided, and it is possible to orient the distal end of the manipulator in a desired direction. As a result, the overtube does not need a driving device, and thus, it is possible to realize good maneuverability by allowing the proximal-end-side tubular portion to be detached without time and effort to perform such work as connecting driving wires or the like.

In the above-described aspect, the distal-end-side tubular portion may be attached to the proximal-end-side tubular portion in a detachable manner.

By doing so, the proximal-end opening of the second channel into which the endoscope is inserted outside the body of a patient is provided at the proximal end of the distal-end-side tubular portion, and it is possible to remove the distal-end-side tubular portion from the proximal-end-side tubular portion at the outside of the body of the patient. In other words, it is also possible to separate the two components in a simple manner by configuring the proximal-end-side tubular portion as a component to be reused and by configuring only the distal-end-side tubular portion as a disposable component.

In addition, the above-described aspect may be provided with a securing portion that secures the endoscope, which is inserted into the second channel, to the distal-end-side tubular portion so as not to move relative to the second channel.

By doing so, it is possible to enhance the maneuverability during treatment by making it easier to make the distal-end-side tubular portion follow the motion of the endoscope by securing the endoscope to the second channel by activating the securing portion.

In addition, in the above-described aspect, the securing portion is configured to secure the endoscope so as not to move in a longitudinal-axis direction of the endoscope relative to the second channel.

By doing so, when the endoscope is moved in the longitudinal-axis direction in the state in which the endoscope and the second channel are secured by using the securing portion, the distal-end-side tubular portion can also be moved in the longitudinal-axis direction of the endoscope, following the motion of the endoscope. In other words, it is possible, by utilizing the rigidity of the endoscope, to perform advance/retract operations even when the rigidity of the overtube is low in the longitudinal-axis direction, and therefore it is possible to easily advance/retract the distal-end position of the manipulator that is positioned at the distal end of the distal-end-side tubular portion.

In addition, in the above-described aspect, the securing portion may be configured to secure the endoscope so as not to move in a rotational direction about a longitudinal axis of the endoscope relative to the second channel.

By doing so, when the endoscope is moved in the rotational direction about the longitudinal axis in the state in which the endoscope and the second channel are secured by using the securing portion, the distal-end-side tubular portion is also rotated about the longitudinal axis of the endoscope, following the motion of the endoscope. In other words, it is possible, by utilizing the torsional rigidity of the endoscope, to perform rotational operation even when the torsional rigidity of the overtube is low in the longitudinal-axis direction, and therefore it is possible to easily rotate the distal-end position of the manipulator that is positioned at the distal end of the distal-end-side tubular portion.

In addition, in the above-described aspect, the distal-end-side tubular portion may be provided with a bending portion at a distal-end portion thereof, and the bending portion has a greater flexibility than the remaining part of the distal-end-side tubular portion.

With this configuration, when the distal-end portion of the endoscope is driven to be bent in the state in which the endoscope is fitted to the second channel, the bending portion of the distal-end-side tubular portion, to which the distal-end portion of the endoscope is fitted, is made to bent in accordance with bending of the endoscope. Because the flexibility thereof is greater than the remaining part of the distal-end-side tubular portion, the bending portion is easily bent, and thus, it is possible to easily move the distal-end position of the manipulator.

In addition, a manipulator system according to another aspect of the present invention is provided with: any one of the above-described overtubes; a manipulator that is inserted into the first channel of the overtube; an endoscope that is inserted into the second channel of the overtube; a driving portion that drives the manipulator; a operating portion that is manipulated by an operator; and a controller that controls the driving portion on the basis of manipulation inputs that are input by using the operating portion.

In addition, the above-described aspect may be provided with a relative movement mechanism that moves the manipulator and the overtube relative to each other in a longitudinal-axis direction of the overtube.

By doing so, it is possible to advance/retract the manipulator with respect to the overtube in the longitudinal-axis direction of the overtube by means of the relative movement mechanism.

### {Advantageous Effects of Invention}

The present invention affords an advantage in which it is possible to detach an overtube from a driving portion without excessive time and effort, in order to achieve a good maneuverability in the overtube.

### {Brief Description of Drawings}

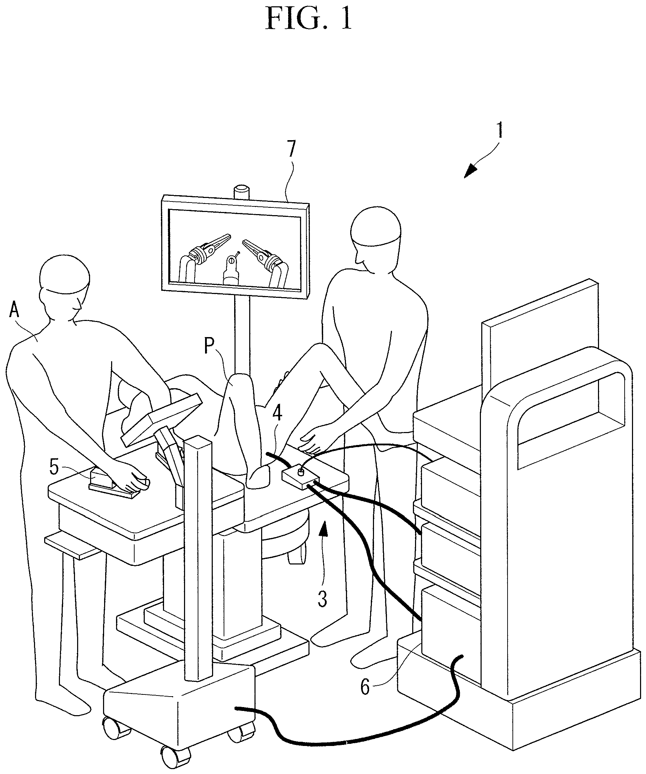

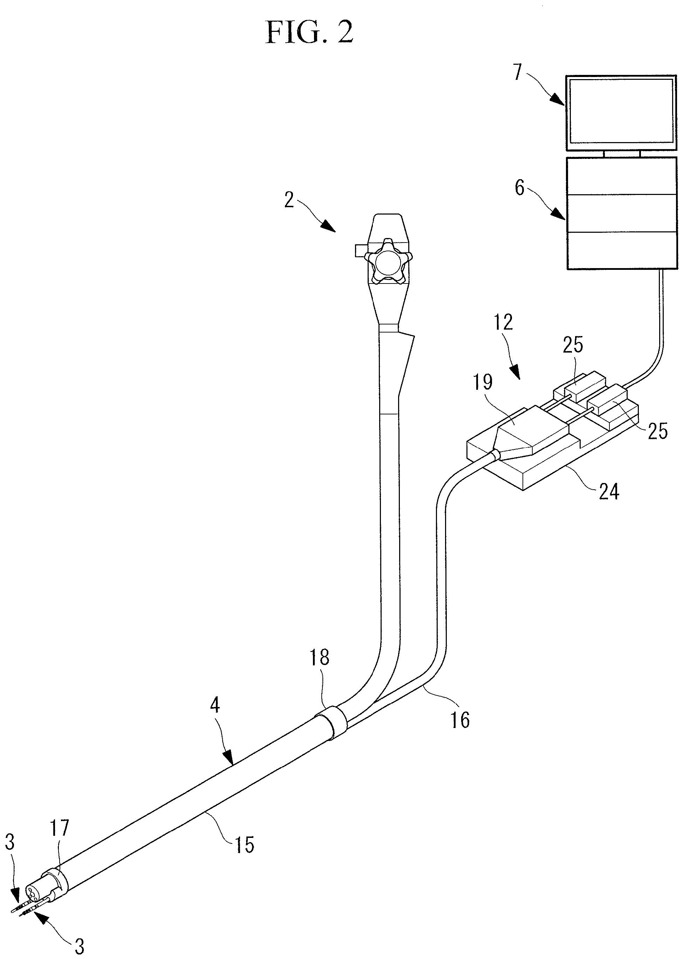

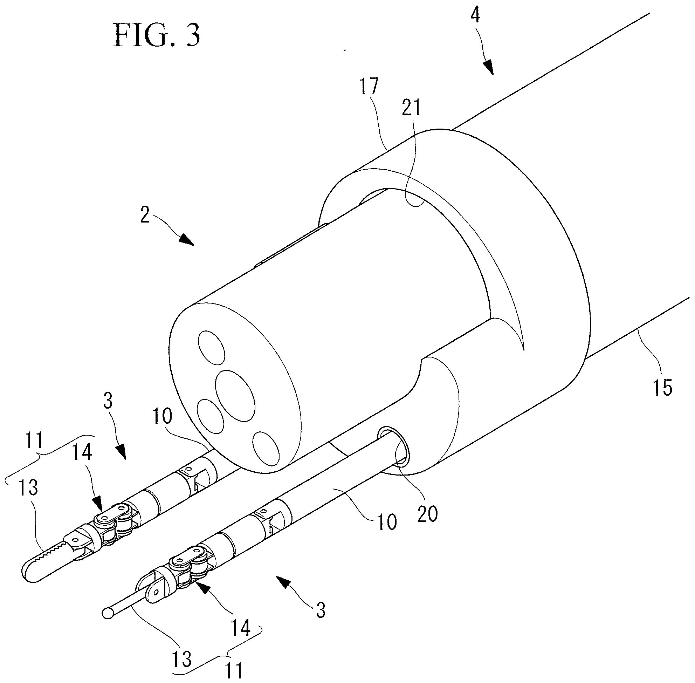

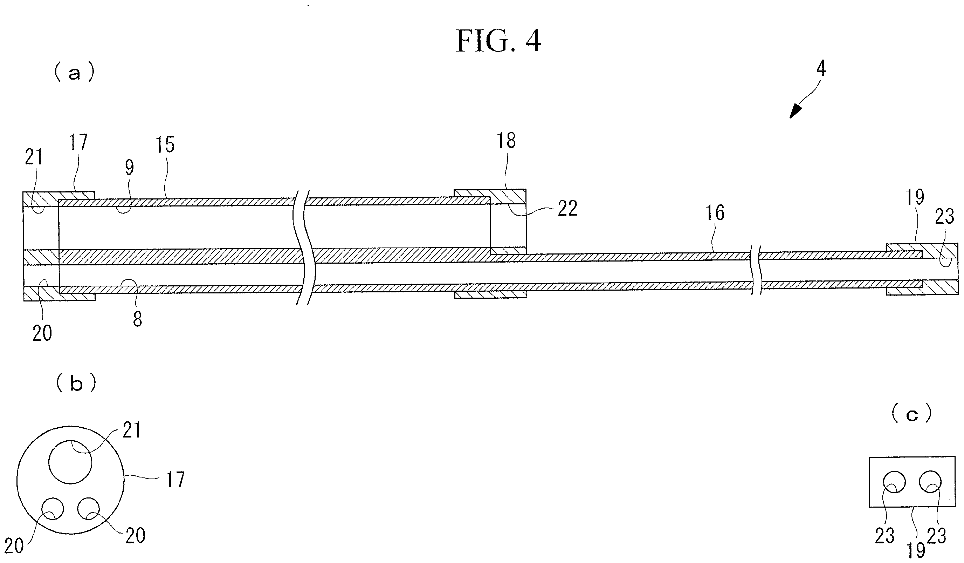

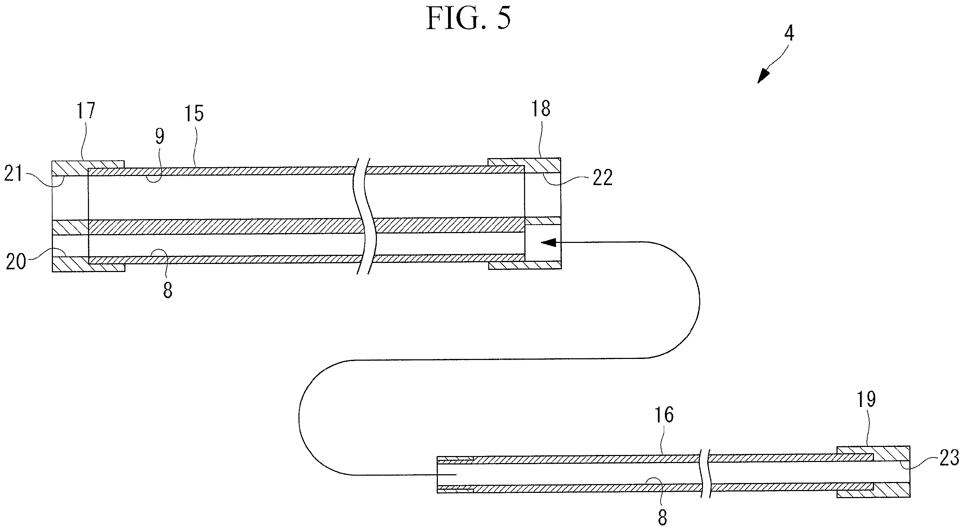

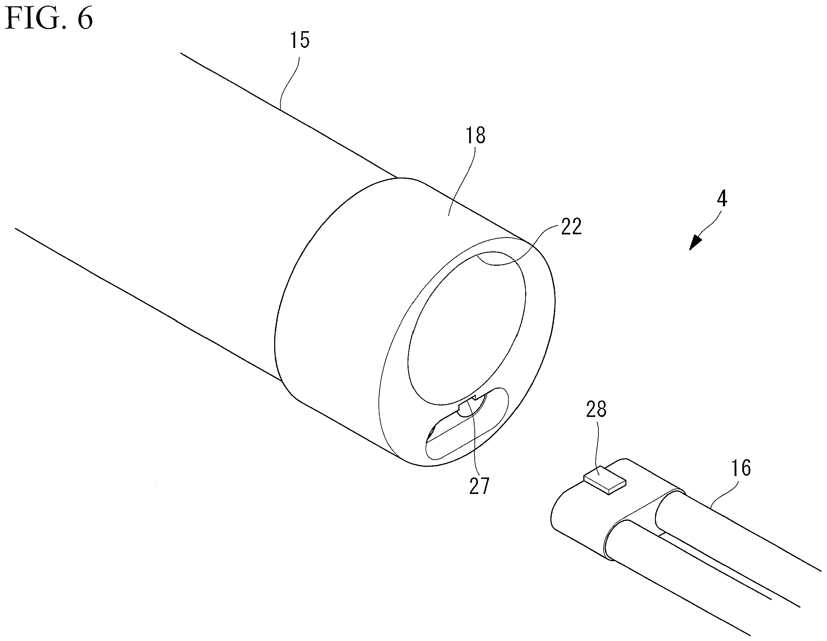

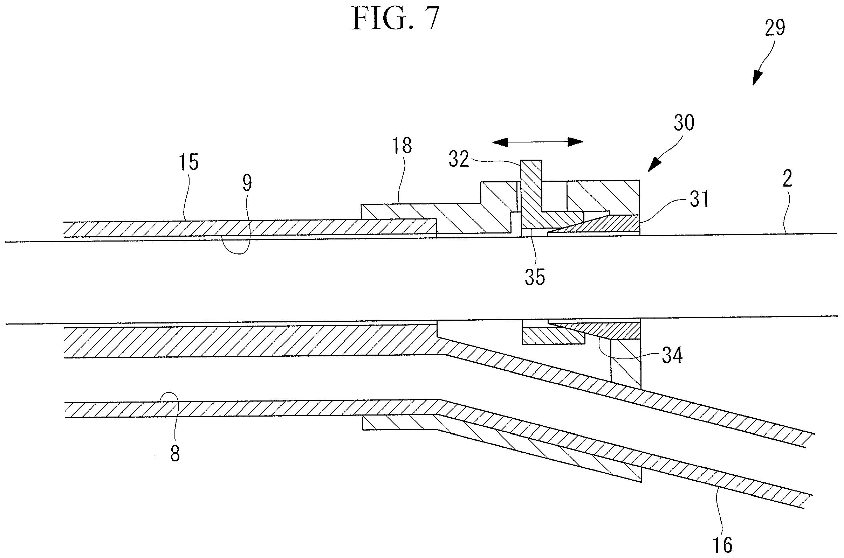

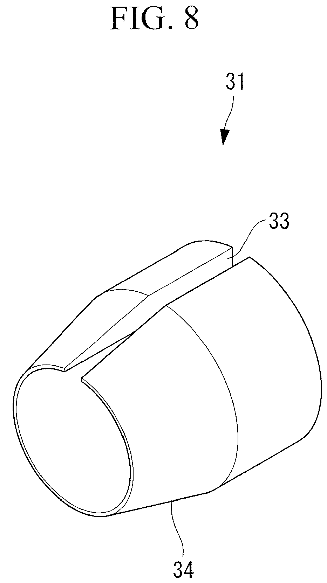

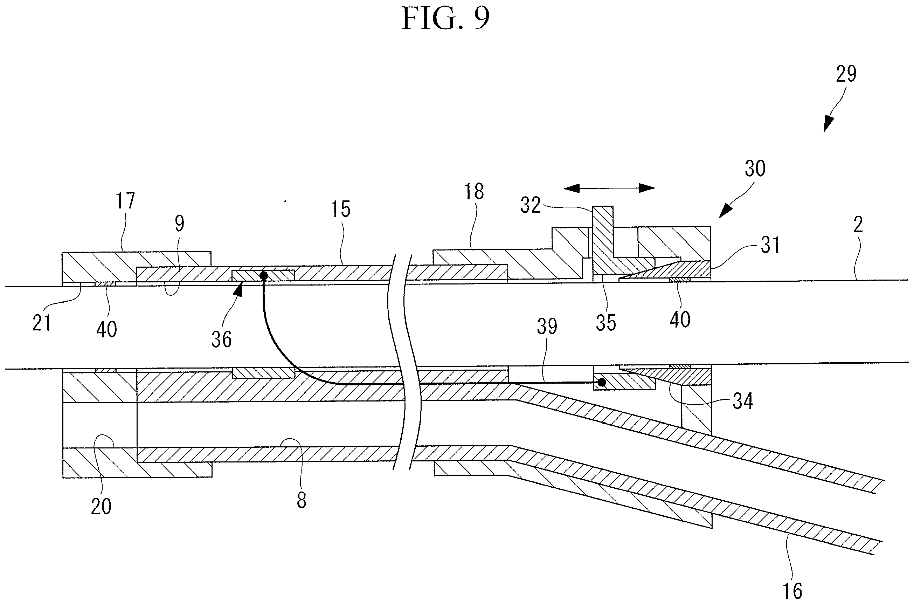

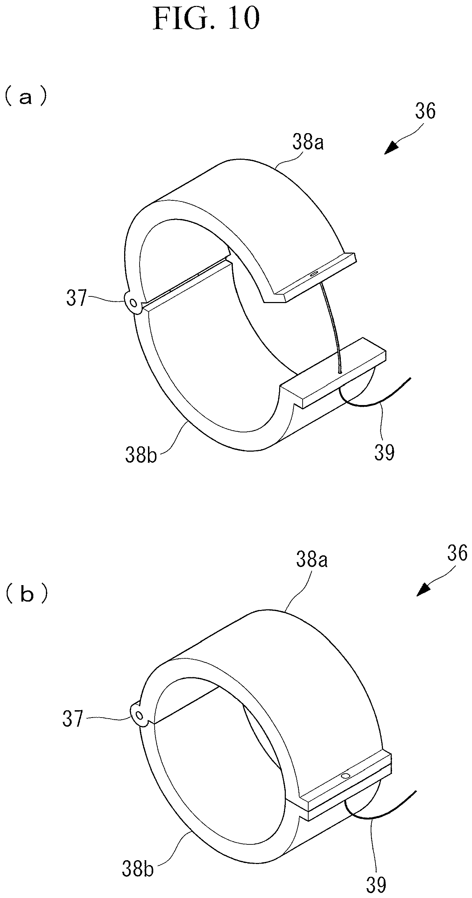

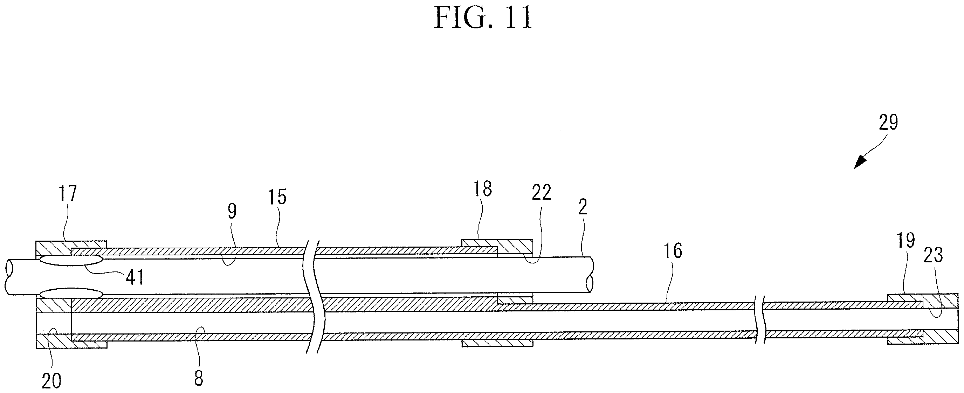

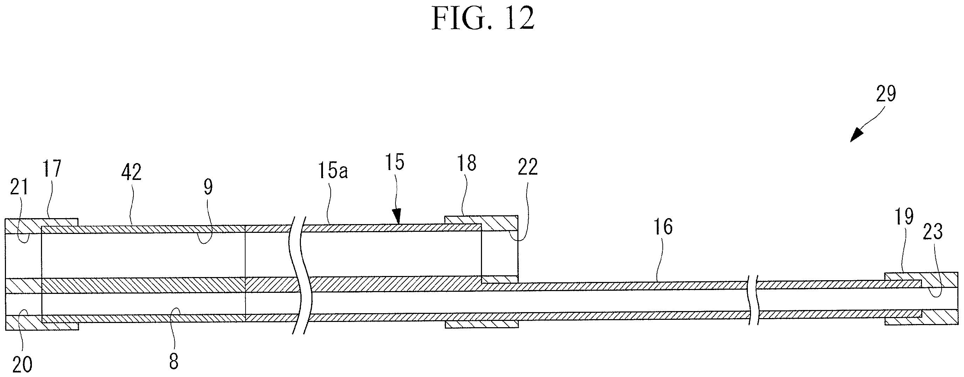

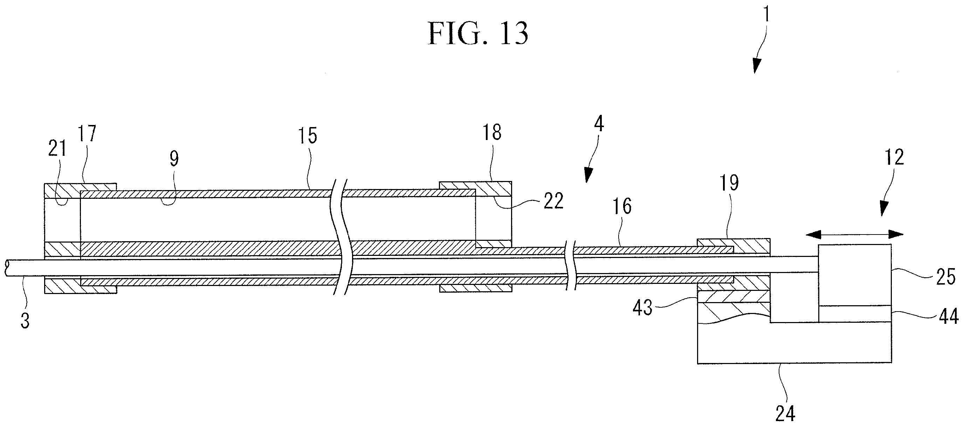

{Fig. 1} Fig. 1 is an overall configuration diagram showing a manipulator system according to a first embodiment of the present invention.
{Fig. 2} Fig. 2 is a perspective view showing the manipulator system in Fig. 1.
{Fig. 3} Fig. 3 is a partial perspective view showing an endoscope, a manipulator, and a distal end of an overtube of the manipulator system in Fig. 2.
{Fig. 4} Fig. 4 shows the overtube according to the first embodiment of the present invention provided in the manipulator system of Fig. 1 in (a) a longitudinal sectional view, (b) a front view of the distal end thereof, and (c) a front view of a proximal end thereof.
{Fig. 5} Fig. 5 is a longitudinal sectional view showing a first modification of the overtube in Fig. 4.
{Fig. 6} Fig. 6 is a partial perspective view showing a connecting portion between a distal-end-side tubular portion and a proximal-end-side tubular portion in a second modification of the overtube in Fig. 4.
{Fig. 7} Fig. 7 is a partial longitudinal sectional view showing the connecting portion between the distal-end-side tubular portion and the proximal-end-side tubular portion in the overtube according to a second embodiment of the present invention.
{Fig. 8} Fig. 8 is a perspective view showing a sleeve provided in the overtube in Fig. 7.
{Fig. 9} Fig. 9 is a partial longitudinal sectional view showing a first modification of the overtube in Fig. 7.
{Fig. 10} Fig. 10 is a perspective view of a second securing portion provided in the overtube in Fig. 9, showing (a) an open state thereof and (b) a closed state thereof.
{Fig. 11} Fig. 11 is a longitudinal sectional view showing a second modification of the overtube in Fig. 7.
{Fig. 12} Fig. 12 is a longitudinal sectional view showing a third modification of the overtube in Fig. 4.
{Fig. 13} Fig. 13 is a partial longitudinal sectional view showing first modifications of the manipulator, the overtube, and a driving portion of the manipulator system in Fig. 1.

### {Description of Embodiments}

An overtube 4 and a manipulator system 1 according to a first embodiment of the present invention will be described below with reference to the drawings.

As shown in Figs. 1 and 2, the manipulator system 1 according to this embodiment is provided with: an endoscope 2 and two manipulators 3 that are inserted into a body of a patient P; the overtube 4 according to an embodiment of the present invention that accommodates them; operating portions 5 that are manipulated by an operator A; a controller 6 that controls the respective manipulators 3 on the basis of manipulations performed via the operating portions 5; and a monitor 7.

As shown in Fig. 3, the manipulators 3 are respectively provided with: inserted portions 10 that are inserted into the body of the patient P via channels 8 and 9 in the overtube 4, which are described later; movable portions 11 that are provided at distal ends of the inserted portions 10; and a driving portion 12 that is disposed on the proximal-end side of the inserted portions 10 and that drives the movable portions 11 by means of driving-power transmitting members (not shown) such as wires or the like.

The movable portions 11 are provided with: treatment portions 13 that are disposed at the most distal ends thereof and that treat an affected site in the body by acting thereon; and a plurality of joints 14 that change positions of distal-end positions of the treatment portions 13 and orientations thereof.

As shown in Fig. 4(a), the overtube 4 according to this embodiment is provided with: a distal-end-side tubular portion 15 that has a tube shape and made of a material possessing flexibility and that has two first channels 8, through which the manipulators 3 are made to pass, and a single second channel 9, through which the endoscope 2 is made to pass; and a proximal-end-side tubular portion 16 that extends from the proximal end of the distal-end-side tubular portion 15 so that the two first channels 8 are extended toward the proximal-end side. A first housing 17 is secured to the distal end of the distal-end-side tubular portion 15, a second housing 18 is secured to the proximal end of the distal-end-side tubular portion 15, and a third housing 19 is secured to the proximal end of the proximal-end-side tubular portion 16.

As shown in Fig. 4(b), the first housing 17 is provided with distal-end openings 20 and 21 from which the distal ends of the two manipulators 3 inserted into the two first channels 8 and the distal end of the endoscope 2 inserted into the second channel 9 are made to protrude, respectively. Note that the first housing 17 may be integrated with the distal-end-side tubular portion 15, and, in this case, the distal-end openings 20 and 21 are integrated with the first channels 8 and the second channel 9, respectively.

The second housing 18 is provided with a proximal-end opening 22 into which the endoscope 2 is inserted.

In addition, as shown in Fig. 4(c), the third housing 19 is provided with two proximal-end openings 23 into which the two manipulators 3 are inserted.

The overtube 4 according to this embodiment is not provided with a movable portion that is moved by means of a driving force. The second channel 9 of the overtube 4 has an inner diameter that is slightly greater than the outer diameter of the endoscope 2, and thus, the endoscope 2 can easily be inserted thereinto in the longitudinal direction. In addition, the distal-end-side tubular portion 15 is configured so that, when a bending portion provided at the distal end of the endoscope 2 is bent, the distal-end side tubular portion 15 is allowed to bend in accordance with the bending thereof.

As shown in Fig. 2, the driving portion 12 of the manipulators 3 is provided with: a driving-portion main body 24 provided with motors; and manipulator-side driving portions 25 that are attached to the driving-portion main body 24 in a detachable manner and that, by being attached to the driving-portion main body 24, transmit the driving forces of the motors to the driving-power transmitting members in the inserted portions 10.

In addition, the third housing 19 of the overtube 4 is configured so as to be attached to the driving-portion main body 24 in a detachable manner by means of an attachable/detachable portion 43 provided between the third housing 19 and the driving-portion main body 24.

An operation of the thus-configured overtube 4 and manipulator system 1 according to this embodiment will be described below.

In order to treat an affected site in a body by using the manipulator system 1 according to this embodiment, the endoscope 2 is made to pass through the overtube 4 in advance, the endoscope 2 is inserted into the body first, and the overtube 4 is subsequently inserted into the body along the endoscope 2. By repeating this procedure, the endoscope 2 and the overtube 4 are inserted to a location at which the affected site exists in the body.

Then, after reaching the peripheral area of the affected site, the distal ends of the two manipulators 3, which are inserted into the first channels 8 from the proximal-end openings 23 provided in the third housing 19, are placed near the distal-end openings 20 of the first housing 17 of the distal-end-side tubular portion 15, and the distal end of the endoscope 2, which is inserted into the second channel 9 from the proximal-end opening 22 provided in the second housing 18, is placed near the distal-end opening 21 of the first housing 17.

Then, in a state in which the distal end of the overtube 4 is placed close to the affected site in the body, the operator A makes the distal end of the endoscope 2 protrude from the distal-end opening 21 of the second channel 9, and makes the distal ends of the two manipulators 3 respectively protrude from the distal-end openings 20 of the first channels 8. In this state, the third housing 19 of the proximal-end-side tubular portion 16 is secured to the driving-portion main body 24, and the manipulator-side driving portions 25 are attached to the driving-portion main body 24.

In a state in which the movable portions 11 at the distal ends of the manipulators 3 are placed in the field of view of the endoscope 2, the operator A manipulates the operating portions 5 while checking images acquired by the endoscope 2 on the monitor 7. The controller 6 controls the motors in the driving-portion main body 24 on the basis of the amount of manipulation input via the operating portions 5 to drive the movable portions 11 of the manipulators 3, and thus, it is possible to treat the affected site.

In this case, if the treatment portions 13 are not appropriately oriented with respect to the affected site, although it is possible to change the orientations of the treatment portions 13 by driving the respective joints 14 of the movable portions 11 of the manipulators 3 by manipulating the operating portions 5, it is possible to change the orientation of the first housing 17 itself by bending the distal-end-side tubular portion 15 of the overtube 4 in accordance with bending of the bending portion of the endoscope 2 by driving the bending portion of the endoscope 2.

Because the first housing 17 is provided with the distal-end opening 21, from which the distal-end portion of the endoscope 2 is made to protrude, and the distal-end openings 20, from which the distal-end portions of the manipulators 3 are made to protrude, it is possible to change the orientations of the movable portions 11 of the manipulators 3 as a whole motion by changing the orientation of the first housing 17.

In other words, with the overtube 4 according to this embodiment, because the orientations of the treatment portions 13 provided in the movable portions 11 of the manipulators 3 are changed by driving the endoscope 2, it is not necessary to provide a driving mechanism which has driving-power transmitting members such as wires or the like in the overtube 4 itself.

Therefore, with the overtube 4 and the manipulator system 1 according to this embodiment, when using the entire overtube 4 as a disposable component, it is not necessary to connect or disconnect the driving mechanism, and, because connection to and removal from other parts are performed without requiring excessive time and effort, there is an advantage in which it is possible to realize good maneuverability.

Note that, in this embodiment, although the overtube 4 in which the distal-end-side tubular portion 15 and the proximal-end-side tubular portion 16 are integrated has been described as an example, alternatively, as shown in Fig. 5, the distal-end-side tubular portion 15 and the proximal-end-side tubular portion 16 may be connected in a detachable manner. Because the second housing 18 that has the proximal-end opening 22 into which the endoscope 2 is inserted is disposed outside the body of the patient P, it is possible to employ a component, which is not inserted into the body of the patient P and that is reused, as the proximal-end-side tubular portion 16 which is disposed farther at the proximal-end-side than the second housing 18.

In this case, it is not necessary to connect or disconnect the driving mechanism when attaching/detaching the distal-end-side tubular portion 15 to/from the proximal-end-side tubular portion 16, and thus, there is an advantage in which it is possible to employ a disposable component only for the distal-end-side tubular portion 15 and to perform connection and separation thereof without excessive time and effort.

In addition, in the case in which the affected site is large and it is necessary to adjust the position of the distal-end position of the overtube 4 in the body of the patient P, the manipulators 3 need to be pulled out of the overtube and to perform manipulation such as pushing, pulling, twisting, or the like of the overtube 4 at the proximal-end side thereof. At this time, such manipulation can be performed without having to swivel the proximal-end-side tubular portion 16 if it is possible to detach the proximal-end-side tubular portion 16 from the distal-end-side tubular portion, and thus, it is possible to realize better maneuverability.

In addition, although the embodiment which has the overtube 4 having the two first channels 8 through which the two manipulators 3 are made to pass is described above, one or three or more first channels 8 may be provided and one or more manipulators 3 may be made to pass therethrough.

In addition, in the case in which the distal-end-side tubular portion 15 and the proximal-end-side tubular portion 16 are connected in a detachable manner, if two or more first channels 8 are provided, it is necessary to make sure the correspondence relationship that the first channels 8 in the distal-end-side tubular portion 15 and the first channels 8 in the proximal-end-side tubular portion 16 are correctly connected. In such a case, as shown in Fig. 6, a depression 27 and a protrusion 28 that fit with each other may be provided at the distal end of the second housing 18 and the distal end of the proximal-end-side tubular portion 16.

Next, an overtube 29 according to a second embodiment of the present invention will be described below with reference to the drawings.

In describing this embodiment, portions having the same configurations as those of the overtube 4 of the above-described first embodiment are given the same reference signs, and descriptions thereof will be omitted.

As shown in Fig. 7, the overtube 29 according to this embodiment is provided, in the second housing 18, with a securing portion 30 that secures the endoscope 2 to the second channel 9. The securing portion 30 is provided with: a sleeve 31 that is secured to the second housing 18 and through which the endoscope 2 is made to pass; and a slider 32 that is provided so as to be movable with respect to the second housing 18 in the longitudinal direction of the second channel 9.

As shown in Fig. 8, the sleeve 31 is configured so as to have a C-shaped cross-section due to a slit 33 formed by cutting out, in the longitudinal direction, a portion at a circumferential-direction position of the sleeve 31, and so that the size thereof can be reduced from a state in which the inner diameter is greater than the outer diameter of the endoscope 2 to a state in which the inner diameter is smaller than the outer diameter of the endoscope 2. In addition, at one end of an outer circumferential surface of the sleeve 31, a tapered surface 34 in which the size thereof decreases toward the distal end is provided. In addition, the slit 33 extends, in the longitudinal direction, from an end portion in the longitudinal direction on the tapered surface 34 side (hereinafter referred to as the one end) to the other end portion (hereinafter referred to as the other end). Alternatively, the slit 33 may extend to an intermediate position in the longitudinal direction from the one end. In other words, it is permissible that the slit 33 does not extend all the way from the one end to the other end in the longitudinal direction, and it is also permissible that the slit 33 does not extend all the way in the thickness direction of the sleeve 31. In addition, a plurality of slits 33 may be provided along the outer circumference of the sleeve 31.

The slider 32 is provided with a fitting hole 35 into which the tapered surface 34 of the sleeve 31 is fitted. The inner diameter of the fitting hole 35 is set to be smaller than the maximum outer diameter of the tapered surface 34 of the expanded sleeve 31 and to be greater than the minimum outer diameter thereof.

With the thus-configured overtube 29 according to this embodiment, when the endoscope 2 is inserted into the second channel 9 from the proximal-end opening 22 of the second housing 18 in a state in which the slider 32 is moved in a direction away from the sleeve 31, the endoscope 2 passes through inside the sleeve 31 and is inserted into the second channel 9. Because the inner diameter of the sleeve 31 is increased by moving the slider 32 in a direction away from the sleeve 31, it is possible to easily insert the endoscope 2 into the second channel 9.

Because the fitting hole 35 of the slider 32 comes into contact with an intermediate position of the tapered surface 34 of the sleeve 31 when the slider 32 is moved in the longitudinal direction of the second channel 9 in this state, by moving the slider 32 further, the fitting hole 35 presses the tapered surface 34, thus compressing the sleeve 31 in the direction in which the slit 33 is narrowed. By doing so, the inner diameter of the sleeve 31 is decreased so as to be smaller than the outer diameter of the endoscope 2, and thus, the endoscope 2 is pressed radially inward by the sleeve 31.

In other words, by gripping the endoscope 2 with the sleeve 31, the endoscope 2 is secured so as not to move in the longitudinal direction and the circumferential direction with respect to the second channel 9. As a result, it is possible to move the overtube 29 in the longitudinal-axis direction together with the endoscope 2 when the operator A presses the endoscope 2 in the longitudinal-axis direction via manipulations thereof performed outside the body of the patient P.

In other words, there is an advantage in which, even if a unit formed of a material having a low rigidity in the longitudinal-axis direction thereof is employed as the overtube 29, it is possible to easily advance/retract the overtube 29 in the longitudinal-axis direction by utilizing the rigidity of the endoscope 2. In addition, there is an advantage in which, even if a unit formed of a material having a low torsional rigidity is employed as the overtube 29, it is possible to easily perform rotation about the longitudinal axis by utilizing the rigidity of the endoscope 2.

Note that, although this embodiment employs the securing portion 30, which secures the endoscope 2 and the second channel 9 at the position of the second housing 18 provided on the proximal end of the distal-end-side tubular portion 15, alternatively, as shown in Fig. 9, it is permissible to employ a configuration in which a second securing portion 36 that is opened/closed in association with advancement/retraction of the slider 32 is provided in the vicinity of the distal end of the distal-end-side tubular portion 15. As the second securing portion 36, for example, as shown in Figs. 10(a) and (b), it is permissible to employ a unit in which two components 38a and 38b that are coupled by means of a hinge 37 so as to be opened/closed are moved in a direction in which they are brought close to each other by means of a wire 39 that is pulled by the slider 32.

By doing so, when the slider 32 is moved, because, not only the securing portion 30 of the second housing 18, but also the endoscope 2 and the second channel 9 are secured by the second securing portion 36 disposed in the vicinity of the distal end of the distal-end-side tubular portion 15, it is possible to easily transmit forces in the longitudinal-axis direction and the circumferential direction applied to the endoscope 2 to the distal end of the overtube 29. As a result, there is an advantage in which it is possible to more easily perform advancing/retracting motions and rotational motions of the overtube 29 and the manipulators 3 by utilizing the rigidity of the endoscope 2.

Although the configuration in which relative movements of the second channel 9 and the endoscope 2 are prohibited, both in the longitudinal-axis direction and the circumferential direction about the longitudinal axis, by using the securing portion 30, alternatively, a configuration in which movements in the longitudinal-axis direction and those in the circumferential direction about the longitudinal axis are prohibited by using separate means may be employed.

In addition, as shown in Fig. 9, sealing members 40 that are brought into close contact with an outer surface of the inserted endoscope 2 in a slidable manner may be disposed at an inner surface of the sleeve 31 provided in the second housing 18 and an inner surface in the vicinity of the distal-end opening 21 of the second channel 9. As has been described above, although the inner diameter of the second channel 9 is formed so as to be slightly greater than the external size of the endoscope 2, because a space therebetween is formed to be small in order to make the distal-end-side tubular portion 15 bend so as to follow bending of the endoscope 2, the friction between the second channel 9 and the endoscope 2 is increased. By providing the sealing members 40, it is possible to decrease the friction by applying a lubricant in the second channel 9 located between the sealing members 40, and it is also possible to prevent the lubricant from leaking out of the second channel 9 by means of the sealing members 40. In addition, the sealing members 40 can also prevent a body fluid from entering the interior of the overtube 4.

In addition, in this embodiment, the endoscope 2 is secured to the second channel 9 by gripping the outer surface of the endoscope 2 with the sleeve 31 by compressing the sleeve 31 in the radial direction by moving the slider 32. Alternatively, as shown in Fig. 11, a balloon 41 that can be inflated and deflated may be disposed in the second channel 9, and the endoscope 2 and the second channel 9 may be secured relative to each other by increasing the friction between the two by inflating the balloon 41 in the state in which the endoscope 2 is inserted into the second channel 9. By disposing the balloon 41 in the vicinity of the distal end of the distal-end-side tubular portion 15, it is possible to easily advance, retract, and rotate the overtube 29 by effectively utilizing the rigidity of the endoscope 2.

In addition, in this embodiment, as shown in Fig. 12, in the distal-end-side tubular portion 15, flexibility may be enhanced, as compared with that of another section 15a, only in a section (bending portion) 42 in the vicinity of the distal end thereof in the second channel 9, at which the bending portion of the endoscope 2 is placed. By doing so, it is possible to make the motion of the distal-end-side tubular portion 15 more easily follow the motion of the bending portion of the endoscope 2.

Note that, in order to enhance the flexibility, it is possible to employ means such as making the tube narrower only in the section 42, using a material having a low flexibility, forming a slit in the tube, or the like.

In addition, in this embodiment, the third housing 19 of the proximal-end-side tubular portion 16 is secured to the driving-portion main body 24, and the manipulator-side driving portions 25 to which the manipulators 3 are connected are attached to the driving-portion main body 24. Alternatively, as shown in Fig. 13, the driving-portion main body 24 may be provided with sliders (relative movement mechanisms) 44 that move the manipulators 3 relative to the overtube 4 in the longitudinal-axis direction thereof, and the manipulator-side driving portions 25 may be attached to the sliders 44 in a detachable manner.

In this case, the sliders 44 may be provided with motors. By doing so, when the sliders 44 are made to slide, the manipulators 3 can be advanced/retracted with respect to the overtube 4 in the longitudinal direction thereof.

In addition, an attachable/detachable portion 43 that attaches the third housing 19 of the overtube 4 to the driving-portion main body 24 in an attachable/detachable manner may be provided so as to be movable with respect to the manipulator-side driving portions 25 in the longitudinal-axis direction of the overtube 4. Furthermore, both of the attachable/detachable portion 43 and the manipulator-side driving portions 25 may be provided so as to be slidable in the longitudinal-axis direction of the overtube 4.

### {Reference Signs List}

- 1: manipulator system
- 2: endoscope
- 3: manipulator
- 4: overtube
- 5: operating portions
- 6: controller
- 8: first channel
- 9: second channel
- 12: driving portion
- 15: distal-end-side tubular portion
- 16: proximal-end-side tubular portion
- 20, 21: distal-end opening
- 22, 23: proximal-end opening
- 30: securing portion
- 42: section in the vicinity of the distal end (bending portion)
- 44: slider (relative movement mechanism)

## Claims

1. An overtube comprising:
a distal-end-side tubular portion that is provided with a first channel through which a manipulator is made to pass and a second channel through which an endoscope is made to pass, and that has flexibility to be driven in accordance with motions of the endoscope; and
a proximal-end-side tubular portion that extends the first channel toward a proximal-end side from a proximal end of the distal-end-side tubular portion,
wherein distal-end openings of the first channel and the second channel are provided at a distal end of the distal-end-side tubular portion,
a proximal-end opening of the second channel is provided at the proximal end of the distal-end-side tubular portion, and
a proximal-end opening of the first channel is provided at a proximal end of the proximal-end-side tubular portion.

2. The overtube according to Claim 1, wherein the distal-end-side tubular portion is attached to the proximal-end-side tubular portion in a detachable manner.

3. The overtube according to Claim 1 or 2, further comprising a securing portion that secures the endoscope, which is inserted into the second channel, to the distal-end-side tubular portion so as not to move relative to the second channel.

4. The overtube according to Claim 3, wherein the securing portion is configured to secure the endoscope so as not to move in a longitudinal-axis direction of the endoscope relative to the second channel.

5. The overtube according to Claim 3 or 4, wherein the securing portion is configured to secure the endoscope so as not to move in a rotational direction about a longitudinal axis of the endoscope relative to the second channel.

6. The overtube according to any one of Claims 1 to 5, wherein the distal-end-side tubular portion is provided with a bending portion at a distal-end portion thereof, and the bending portion has a greater flexibility than the remaining part of the distal-end-side tubular portion.

7. A manipulator system comprising:
an overtube according to any one of Claims 1 to 6;
a manipulator that is inserted into the first channel of the overtube;
an endoscope that is inserted into the second channel of the overtube;
a driving portion that drives the manipulator;
a operating portion that is manipulated by an operator; and
a controller that controls the driving portion on the basis of manipulation inputs that are input by using the operating portion.

8. The manipulator system according to Claim 7, further comprising a relative movement mechanism that moves the manipulator and the overtube relative to each other in a longitudinal-axis direction of the overtube.
